# EUROPEAN PATENT APPLICATION

(11) **EP 2 348 037 A1**
(43) Date of publication of application: **27.07.2011**
(21) Application number: 09819275.0
(22) Date of filing: 09.10.2009
(51) Int. Cl.: C07K 1/08, A61K 49/00, A61K 51/00, C07K 14/645, C07K 14/655, C07K 16/00

(54) **METHOD FOR INTRODUCING DOTA**

(30) Priority: 10.10.2008 JP 2008264520
(71) Applicant: Riken, Wako-shi, Saitama 351-0198 (JP)
(72) Inventor: HASEGAWA, Koki, Kobe-shi Hyogo 650-0047 (JP); WATANABE, Yasuyoshi, Kobe-shi Hyogo 650-0047 (JP)
(74) Representative: Duckworth, Timothy John
(86) International application number: PCT/JP2009/067647
(87) International publication number: WO 2010/041742

(57) **Abstract**

Provided is a convenient, efficient, cost-effective method for introducing 1,4,7,10-tetraazacyclododecane-N,N',N",N"'-tetraacetic acid (DOTA) into a compound such as a peptide.

A method of introducing DOTA into a compound, comprising a first step for preparing a mixed liquid of DOTA having no protecting group and dimethylsulfoxide, and a second step for contacting the mixed liquid with the compound carried on a solid-phase carrier.

## Description

### Technical Field

The present invention relates to a method of synthesizing a compound having a DOTA moiety.

### Background Art

In diagnostic methods such as nuclear magnetic resonance imaging (MRI) and nuclear medicine imaging, or therapies involving the use of radioactive pharmaceuticals, a wide variety of radioactive metals are utilized. For these purposes, radioactive metals are administered to living organisms; to this end, it is necessary to reduce the high toxicities of the radioactive metals by forming complexes with appropriate chelating agents. A wide variety of substances have been developed as such chelating agents; representative examples include 1,4,7,10-tetraazacyclododecane-N,N',N",N"'-tetraacetic acid (DOTA) and diethylenetriaminepentaacetic acid (DTPA). Thereof, DOTA, in particular, is known not only to be suitable for utilization in diagnostic contrast media, but also to surpass DTPA in-utility for radioactive pharmaceuticals because it has the characteristic of forming a thermodynamically and kinetically extremely stable complex with metal ions such as of lanthanoids.

To deliver radioactive metals to target sites in the body, an appropriate targeting vector that interacts selectively with a particular cell type, the endocrine system and the like in living organisms is coupled to DOTA. The targeting vector is typically one of bioactive substances such as peptides, monoclonal antibodies, proteins, oligonucleotides, and glycoproteins, of which peptides, in particular, are highly potential because they have advantages such as good profiles of pharmacokinetics and tumor permeability. For example, somatostatin incorporating DOTA introduced thereinto (DOTA-somatostatin conjugates) has been developed and investigated to diagnose and/or treat somatostatin receptor-positive tumors by selectively delivering radioactive metals to tumor cells or the endocrine system, where somatostatin receptors are expressed at high density.

Regarding methods of introducing (conjugating) DOTA into compounds (e.g., peptide), various investigations have been conducted to date, which can be roughly divided into methods performed in liquid phases (liquid-phase conjugation) and methods performed in solid phases (solid-phase conjugation). Thereof, liquid-phase conjugation necessitates removing excess reaction reagents and by-products to purify the product at every stage of the reaction, which in turn results in decreased yields of the finished product. In contrast, solid-phase conjugation is such that compound molecules are coupled onto a carrier such as beads, and the carrier is placed in a solution of reaction reagents and subjected to a step-by-step synthetic reaction, so that elimination of excess reagents can be achieved merely by filtering the solid phase resin, and the formation of by-products is also suppressed. Also, methods of solid phase synthesis of peptides and the like have been established; by combining such a method and solid-phase conjugation of DOTA, synthesis of a DOTA-incorporating compound (e.g., DOTA-peptide conjugate) can be achieved efficiently.

However, to efficiently perform solid-phase conjugation in combination with a method of solid phase synthesis of compound, various technical problems remain to be overcome. Most importantly, the ligand containing a DOTA group, to be subjected to the conjugation reaction with the compound, should match the chemical conditions used for the method of solid phase synthesis of compound and be soluble. Means that meet these requirements include the tris-tert-butyl ester of DOTA. This is currently commercially available, and is most commonly used to introduce DOTA into a peptide on a solid-phase carrier. Although this is certainly useful because it is freely soluble in most organic solvents, and also because protection in the form of the tert-butyl ester completely matches ordinary techniques of solid phase synthesis, it involves drawbacks such as long times taken to remove the protecting group (2 to 14 hours; see Non-patent Document 1). To overcome these problems, various DOTA derivatives have been developed, but no satisfactory solution has been arrived at with respect to the time requirement for deprotection, and the derivatives remain insufficient to ensure practical use because of the complexity of synthesis, cost-related problems and the like (see Non-patent Document 2). Also, because DOTA having no protecting group is only slightly soluble in organic solvents, it has been thought to be unusable in methods of solid phase synthesis that do not permit the use of an aqueous solution.

### Prior Art Documents

### Non-patent Documents

non-patent document 1: Tetrahedron Letter, vol. 45, p.5453-5455 (2004)
non-patent document 2: Bioconjugate Chemistry, vol. 19, p.391-402 (2008)

### Summary of the Invention

### Problems to Be Solved by the Invention

As is seen from the above-described present situation in the relevant technical field, there is a demand for a convenient, efficient, cost-effective method for introducing DOTA into compounds such as peptides. It is an object of the present invention to provide such a method.

### Means for Solving the Problems

The present inventors succeeded in introducing DOTA into a peptide by mixing DOTA having no protecting group, which had been thought to be only slightly soluble in organic solvents, with dimethylsulfoxide (hereinafter also referred to as "DMSO"), activating the DOTA in the mixed liquid, and contacting and reacting the activated DOTA with a resin for solid phase synthesis carrying the peptide. When DOTA is thus introduced into a peptide, a desired compound can be obtained merely by removing the protecting group from the peptide, and cutting out the compound from the resin for solid phase synthesis, in the same manner as the ordinary procedures in solid phase peptide synthesis, because the DOTA does not have a protecting group. Hence, the present inventors found that it is possible to very conveniently introduce DOTA into a compound such as a peptide by this method, and have developed the present invention.

Accordingly, the present invention is as follows:
[1] A method of introducing 1,4,7,10-tetraazacyclododecane-N,N',N",N"'-tetraacetic acid (DOTA) into a compound, comprising a first step for preparing a mixed liquid of DOTA having no protecting group and dimethylsulfoxide, and a second step for contacting the mixed liquid with a compound carried on a solid-phase carrier.
[2] The method described in [1] above, wherein the first step comprises a treatment for activating the DOTA using an activator.
[3] The method described in [1] or [2] above, wherein the compound is a peptide or a polynucleotide.
[4] The method described in [3] above, wherein the compound is a peptide.
[5] The method described in [4] above, wherein the peptide is an antibody.
[6] The method described in [4] above, wherein the peptide is at least one of somatostatin, secretin, octreotide, exenatide and QBP1.
[7] The method described in [3] above, wherein the compound has been synthesized on the solid-phase carrier.
[8] The method described in any one of [1] to [7] above, wherein the activator is 1-ethyl-3-(dimethylaminopropyl)carbodiimide.

### Effect of the Invention

The method of the present invention makes it possible to conveniently and efficiently introduce DOTA into a compound (e.g., peptide and the like) on a solid-phase carrier. In particular, in the method of the present invention, DOTA having no protecting group is used, so that the time required for final deprotection decreases significantly, compared with the conventional method, which involves the use of DOTA having a protecting group. Also, according to the method of the present invention, DOTA can easily be introduced into a peptide carried on a solid-phase carrier synthesized by solid phase peptide synthesis (SPPS), which is a standard technique in peptide synthesis, so that the present invention provides a highly practical method of synthesizing a DOTA-peptide conjugate. Furthermore, DOTA having no protecting group is much less expensive (about 1/10) compared with DOTA having a protecting group, allowing a significant cost reduction in the synthesis of a DOTA-incorporating compound.

### Modes for Embodying the Invention

The present invention provides a method of introducing DOTA into a compound, comprising a first step for preparing a mixed liquid of DOTA having no protecting group and dimethylsulfoxide, and a second step for contacting the mixed liquid with the compound carried on a solid-phase carrier.

The DOTA having no protecting group used in the method of the present invention is 1,4,7,10-tetraazacyclododecane-N,N',N",N"'-tetraacetic acid, which is a compound represented by the following formula:

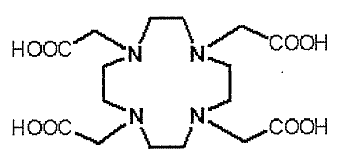

DOTA can be synthesized according to methods described in literature documents, which include Bioconjugate Chemistry, vol.12, p.7-34 (2001) and the like. Alternatively, the DOTA used may be one publicly commercially available from Macrocyclics Company and the like.

Herein, "introducing DOTA into a compound" means subjecting DOTA and the compound to a condensing reaction to synthesize a substance comprising DOTA and the compound which are bound together. The condensation may be, for example, condensation between the carboxyl group of DOTA and the amino group of the compound, or condensation between the carboxyl group of DOTA and the hydroxyl group of the compound. Also, as is obvious in the relevant technical field, the binding of DOTA and compound can take place in various positions in the compound (e.g., N-terminus of the peptide compound, lysine residue in the compound, and the like); in the method of the present invention, the binding position of DOTA and the compound is not particularly limited. The method of the present invention, as described below, can be suitably applied to, for example, peptide compounds; in this case, from the viewpoint of the ease of introduction and compound preparation, DOTA is normally introduced into the N-terminus of the peptide compound. Also, it is preferable that DOTA be activated using an activator or otherwise before being contacted with the compound to have DOTA introduced thereinto. Activation of DOTA is described below.

"Contact" in the above-described second step is not particularly limited, as far as it is a treatment that allows a chemical reaction to be caused between DOTA and the compound to have DOTA introduced thereinto. For example, a treatment wherein the mixed liquid obtained in the above-described first step and a solid-phase carrier carrying the compound are placed and stirred in a container, a treatment wherein a solid-phase carrier carrying the compound is packed in a column, to which the mixed liquid obtained in the above-described first step is fed, and the like can be mentioned. Also, the mixed liquid may be dripped or fluidized onto a substrate wherein a solid-phase carrier layer carrying the compound is formed.

The compound to have DOTA introduced thereinto may be an optionally chosen compound, as far as the compound has a functional group (e.g., amino group, hydroxyl group) that can be utilized for the above-described condensing reaction, and can be carried on a solid-phase carrier; such a compound is chosen as appropriate according to the intended use. Such compounds can be, for example, peptides, polynucleotides, polysaccharides, low-molecular organic compounds and the like, and are suitably peptides.
Also, compounds that do not have a functional group (e.g., amino group, hydroxyl group) that can be utilized for the above-described condensing reaction can also be made to be compounds to which the method of the present invention is applicable, by introducing, for example, an amino group or a hydroxyl group, using a publicly known method, in advance. For example, the amino acid phenylalanine has no amino group in the side chain thereof; however, by introducing an amino group into the side chain using a coupling agent or the like to convert to aminophenylalanine, it becomes possible to introduce DOTA into the side chain by the method of the present invention.
The compound described above is not subject to limitations with respect to how to acquire the same, as far as it is carried on a solid-phase carrier at the stage of introducing DOTA; for example, the compound may be a compound synthesized by an organic synthetic chemical method (e.g., methods of solid phase synthesis (e.g., solid phase peptide synthesis), methods of liquid phase synthesis and the like), a compound synthesized by a biotechnological method (e.g., gene engineering-based method of synthesis and the like), a compound obtained from nature by a publicly known method of isolation and purification and the like.

The method of the present invention is also applicable to compound libraries generated using combinatorial chemistry technology, more specifically to, for example, compound libraries (e.g., peptide libraries, antibody libraries and the like) generated by methods of solid phase synthesis (e.g., solid phase peptide synthesis), the phage display method and the like, on a solid phase. This library may be one prepared in house, or may be a publicly commercially available one.
Herein, "a compound library" means an assembly of discrete compounds carried on an appropriate solid-phase carrier; here, the solid-phase carrier is not particularly limited, as far as it allows DOTA to be introduced into the group of compounds by the method of the present invention; the compound is typically, but is not limited to, a peptide or an antibody, and the assembly comprises typically 1 to about 100000 kinds of compounds, preferably 2 to about 10000 kinds of compounds, more preferably 2 to about 1000 kinds of compounds, still more preferably 5 to about 100 kinds of compounds, as constituents thereof. Methods of generating a compound library are publicly known in the relevant technical field; a compound library that can be used in the method of the present invention may be one generated by any method. Methods of generating a compound library are described in, for example, "Combinatorial Chemistry - Nyumon Kara Ouyou Made (published by Kagaku-Dojin Publishing Co., Ltd.) (1997)" and elsewhere.

Herein, "peptides" broadly mean substances wherein two or more amino acids are joined via amide bonds, and include substances normally called proteins, wherein several tens to several hundred or several thousand amino acids are joined. Examples of the peptide to have DOTA introduced thereinto by the method of the present invention include ones synthesized by a publicly known method of solid phase peptide synthesis (SPPS); in this case, the peptide normally contains 2 to about 100 amino acids. Also, amino acids that constitute the peptide include naturally occurring L-amino acids, naturally occurring D-amino acids, and non-naturally-occurring amino acids (including not only publicly known compounds such as phenylglycine and p-hydroxyphenylglycine, but also optionally chosen compounds having an amino group and a carboxyl group, derivatized from amino acids). Furthermore, substances wherein a sugar and/or a lipid is bound to a substance having two or more amino acids joined via amide bonds are also encompassed in "peptides" as mentioned herein. Specific examples of the peptide include somatostatin, secretin, octreotide, exenatide, QBP1 (Polyglutamine binding peptide 1) and the like.
Methods for synthesizing a peptide by organic synthetic chemistry (e.g., methods of solid phase synthesis, methods of liquid phase synthesis and the like), and methods for biotechnologically synthesizing a peptide (e.g., gene engineering-based methods of synthesis and the like) have already been established in the relevant technical field, and methods of peptide isolation and purification have also been established. Furthermore, various instruments for such operations (e.g., automated solid phase peptide synthesizers and the like), kits and the like are publicly commercially available. Peptides utilized for the method of the present invention can be obtained using these known methods and means.

Also, DOTA-incorporating antibodies (DOTA-antibody conjugates) are currently in use for diagnostic and therapeutic applications; therefore, as a preferable example of peptides to which the method of the present invention is applicable, antibodies can be mentioned.
Herein, "antibodies" mean immunoglobulins (IgA, IgD, IgE, IgG, and IgM, and Fab fragments, F(ab')₂ fragments, and Fc fragments thereof); examples include polyclonal antibodies, monoclonal antibodies, single-chain antibodies, anti-idiotype antibodies, humanized antibodies and the like, with preference given to monoclonal antibodies. Preparation of an antibody can be achieved using various methods known per se.
Specific examples of the antibody to have DOTA introduced thereinto using the method of the present invention include, but are not limited to, cetuximab and the like.

Herein, "polynucleotides" broadly mean substances wherein two or more nucleotides are joined, and may be single-stranded or double-stranded, and may be polydeoxynucleotides or polyribonucleotides. Also, chimeras of DNA and RNA, peptide nucleic acids and the like are also included in "polynucleotides" as mentioned herein. Regarding the length of polynucleotides, relatively short (typically 50 bases or less) ones normally called oligonucleotides are also encompassed, and longer DNA, RNA and the like are also encompassed. The polynucleotide may comprise a modified base, for example, a base that does not occur in nature, such as inosine, or a tritylated base and the like. Short polynucleotides (normally 2 to about 50 bases) can be chemically synthesized by known methods, for example, the triester method, the phosphite method, the phosphoramidite method, the phosphonate method and the like. Such a synthesis can normally be carried out on a modified solid support; automated synthesizers for this purpose are publicly commercially available. The polynucleotide to have DOTA introduced thereinto by the method of the present invention is suitably a polynucleotide synthesized on a solid-phase carrier by a method of solid phase synthesis having a length of 2 to about 50 bases.

The above-described first step can be performed by simply mixing DOTA having no protecting group and dimethylsulfoxide. Because DOTA is only slightly soluble in DMSO, a suspension is normally obtained by the above-described step. Also, as stated above, the above-described first step preferably comprises a treatment for activating DOTA using an activator. Herein, examples of "a treatment for activating DOTA" include a treatment wherein DOTA is reacted with an appropriate active esterifying component (e.g., N-hydroxyamines such as succinimide, phenols) to form a reactive ester and the like. Here, "an active esterifying component" means a substance capable of rendering DOTA in an activated state by forming an ester with DOTA. The activated DOTA can be used to cause the above-described condensing reaction with a compound to introduce DOTA into the compound.

Preparation of DOTA activated in a mixed liquid with DMSO can be achieved, for example, as described below.
That is, after an active esterifying component (e.g., N-hydroxyamines such as succinimide, phenols), an activator, and DOTA are added to DMSO, the mixture may be stirred for an appropriate time (e.g., 3 to 6 hours) to carry out the activating reaction. Here, the order of adding the individual compounds is not particularly limited; the reaction can be carried out at, for example, room temperature. Also, "an activator" means a substance for promoting the esterifying reaction of DOTA and the above-described active esterifying component. The activator used may be an optionally chosen one, as far as the desired goal can be attained; however, from the viewpoint of the ability to give a high percent yield when used in DMSO, and the ability to suppressing the generation of unwanted by-products that interfere with the reaction, it is preferable to use a water-soluble carbodiimide such as 1-ethyl-3-(dimethylaminopropyl)carbodiimide. When the step is carried out using 1-ethyl-3-(dimethylaminopropyl)carbodiimide, succinimide, DOTA and DMSO, the above-described activating reaction can be carried out by, for example, adding 10 µmol of 1-ethyl-3-(dimethylaminopropyl)carbodiimide, 10 µmol of succinimide and 10 µmol of DOTA to 1 mL of DMSO, and stirring the mixture at room temperature for 3 hours.

Next, the mixed liquid obtained in the above-described first step is contacted with a solid-phase carrier carrying a compound to introduce DOTA into the compound. For this purpose, it is necessary to prepare a solid-phase carrier carrying the compound in advance. As stated above, the compound may be one obtained from nature or synthesized by an appropriate method of synthesis, and immobilized on a solid-phase carrier by an optionally chosen publicly known method, or may be synthesized on a solid-phase carrier by a method of solid phase synthesis.

Herein, "a compound carried on a solid-phase carrier" means a compound that is immobilized on the surface of an appropriate solid-phase carrier as described below by a certain physical or chemical means, and that is capable of remaining immobilized on the solid-phase carrier during the step of introduction (conjugation) of DOTA. The means may be an optionally chosen method publicly known in the relevant technical field; examples include, but are not limited to, a covalent bond with the compound that constitutes the solid-phase carrier, a biotin-avidin bond, a biotin-streptavidin bond and the like. For example, a peptide synthesized by a standard method of solid phase peptide synthesis is finally cut out from the solid-phase carrier by an appropriate treatment; the peptide before being cut out is "a compound carried on a solid-phase carrier" as mentioned in the present invention. Alternatively, a method of immobilizing an antibody on an appropriate solid-phase carrier such as beads is publicly known in the relevant technical field; an antibody immobilized on a solid-phase carrier by such a method is also "a compound carried on a solid-phase carrier" as mentioned in the present invention.

Herein, "a solid-phase carrier" means an inert material capable of carrying a compound to have DOTA introduced thereinto directly or via a binding arm. A solid-phase carrier can be chosen as appropriate from among all materials in common use for binding peptides, polynucleotides, polysaccharides, organic low-molecular compounds and the like in the relevant technical field. Examples of solid-phase carriers include, but are not limited to, resins such as chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, and 4-(2',4'-dimethoxyphenyl-Fmoc aminoethyl)phenoxy resin, as well as polymers such as polycarbonate, polyethylene, polypropylene, polystyrene, saturated cyclic polyolefin, polypentene, polyamide, and copolymers thereof, and the like.
Also, the solid-phase carrier may be in an optionally chosen form such as a tabular substrate, microwell plates, or microbeads and the like, as far as the carrier can be used in the method of the present invention.

A mode of embodiment of the present invention is a method wherein DOTA is introduced into a peptide on a solid-phase carrier synthesized by a publicly known method of solid phase peptide synthesis. Because DOTA-peptide conjugates are important in diagnostics and therapeutics, also because methods of solid phase peptide synthesis have already been established well, and still also because one of the features of the present invention, i.e., introduction of DOTA on a solid-phase carrier, is efficiently utilized, and for other reasons, such modes of embodiment are highly useful. For this reason, such modes of embodiment, in particular, are hereinafter described in detail. However, the present invention is not limited to such modes of embodiment; it is evident that those skilled in the art are able to obtain a desired substance incorporating DOTA introduced thereinto, on the basis of the description below, using, for example, a method of solid phase oligonucleotide synthesis, a method of solid phase polysaccharide synthesis or the like, which are publicly known in the relevant technical field, or by utilizing a publicly known technique for immobilizing the above-described compound into which DOTA can be introduced by the method of the present invention, on a solid-phase carrier.

Synthesis of a peptide by a method of solid phase synthesis is described below. The condensation and protecting group detachment described below can be achieved according to, for example, methods described in (1) to (5) below and elsewhere:
(i) M. Bodanszky and M.A. Ondetti: Peptide Synthesis, Interscience Publishers, New York (1966)
(ii) Schroeder and Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975);
(iv) Haruaki Yajima and Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten (1991).
In the method of the present invention, it should be noted that the detachment of the protecting group from the peptide synthesized on the solid-phase carrier, and the cutting out of the peptide from the solid-phase carrier, are performed after the introduction of DOTA described below.

In the method of the present invention, optionally chosen methods of solid phase peptide synthesis in common use in the relevant technical field can be applied; the Fmoc method and the Boc method are preferred, with greater preference given to the Fmoc method. Furthermore, there are two methods of synthesis for the Fmoc method: the batch method, wherein a resin and amino acids are placed and stirred in a container to cause the reaction, and the continuous flow method, wherein a resin is packed in a column, to which amino acids are fed to cause the reaction; although both can be used, the batch method can be used suitably.

The carrier resin used in these methods of solid phase synthesis may be any carrier resin for methods of solid phase synthesis that is commercially available in a form incorporating a C-terminal amino acid derivative introduced thereinto in advance can be used preferably. Regarding such carrier resins, carrier resins used in the Fmoc method, for example, include, but are not limited to, Wang resin and 2-chlorotrityl resin (these are for the batch method); KA resin and PA resin (these are for the continuous flow method); TGT resin and TGA resin (these are for the batch method and the continuous flow method). Also, resins used for the Boc method include, but are not limited to, Merrifield resin, PAM resin and the like. Regarding all of the carrier resins exemplified above, products incorporating various amino acid derivatives introduced thereinto to allow them to be applied to optionally chosen peptides to be synthesized are available from, for example, Calbiochem-Novabiochem Japan K.K. By condensing amino acids having the α-amino group and side-chain functional group protected as appropriate, on such a resin, according to the sequence of the desired peptide, by various methods of condensation known per se, a solid-phase carrier carrying the desired peptide can be obtained.

Regarding the above-described condensation of protecting amino acids, various activating reagents usable for peptide synthesis can be used, with particular preference given to carbodiimides. Carbodiimides include dicyclohexylcarbodiimide (DCC), N,N'-diisopropylcarbodiimide, N-ethyl-N'-(3-dimethylaminopropyl)carbodiimide and the like; N-ethyl-N'-(3-dimethylaminopropyl)carbodiimide is preferably used. In the activation therewith, protected amino acids, along with a racemation suppressing additive (for example, HOBt, HOOBt), may be added directly to the resin, or may be added to the resin after the protected amino acid, as a symmetric acid anhydride or as a HOBt ester or HOOBt ester, is activated in advance.
In the method of the present invention, the solvent used to activate the protected amino acid and condense with the resin is normally DMSO. A reaction temperature is chosen as appropriate over a range known to be usable for peptide-bond forming reactions, and is normally chosen as appropriate over the range of about -20°C to 50°C. The amino acid derivative thus activated is normally used in 1.5- to 4-fold excess. If a test using a ninhydrin reaction yields a result suggestive of insufficient condensation, sufficient condensation can be achieved by repeating the condensing reaction. If no sufficient condensation is achieved even by repeating the reaction, it is possible to prevent the subsequent reaction from being influenced, by acetylating the unreacted amino acids using acetic anhydride or acetylimidazole.

After completing the preparation of the peptide-carrying solid-phase carrier, a step for introducing DOTA into the peptide is carried out using the mixed liquid obtained in the above-described first step.
For the sake of this step, the condensing reaction may be carried out by, for example, adding to the compound-carrying solid-phase carrier the above-described DMSO suspension and, for example, a solvent for swelling the solid-phase carrier, such as N-methylpyrrolidone, a reaction promoter such as diisopropylamine, and the like, and stirring the mixture at, for example, room temperature, for an appropriate time (e.g., 1 to 2 hours). If a test using a ninhydrin reaction yields a result suggestive of insufficient condensation, sufficient condensation can be achieved by repeating the condensing reaction. If no sufficient condensation is achieved even by repeating the reaction, it is possible to prevent the subsequent reaction from being influenced, by acetylating the unreacted amino acids using acetic anhydride or acetylimidazole.
After completion of the condensation step, the solid-phase carrier after the reaction is washed with methanol and the like, and dried under reduced pressure.

To obtain a desired compound (DOTA-peptide conjugate), it is necessary to treat the solid-phase carrier, dried under reduced pressure as described above, to eliminate the protecting group in the compound and cut out the compound from the solid-phase carrier. The method involving this step can be performed as chosen as appropriate from among the methods performed in ordinary methods of solid phase peptide synthesis according to the choice of the amino acid protecting group used in the solid phase peptide synthesis and the mode of the binding of the solid-phase carrier and the compound. The target compound thus having the protecting group removed and cut out may be isolated and purified by a publicly known method of purification, whereby the desired compound can be obtained. Here, methods of purification include, for example, solvent extraction, distillation, column chromatography, liquid chromatography, recrystallization, combinations thereof and the like.

How to acquire a DOTA-peptide conjugate, in particular, has been described in detail above; those skilled in the art are able to obtain other compounds (e.g., polynucleotides, polysaccharides and the like) having DOTA introduced thereinto, with reference to the above description, by utilizing techniques obvious in the relevant technical field.

### Examples

The present invention is hereinafter described in further detail by means of the following Examples, by which, however, the present invention is not limited in any way.

### [Example 1]

2 mg of succinimide and 4 mg of 1-ethyl-3-(dimethylaminopropyl)carbodiimide were dissolved in 2 mL of DMSO, 8 mg of 1,4,7,10-tetraazacyclododecane-N,N',N",N"'-tetraacetic acid (DOTA) was added thereto, and this was followed by stirring at room temperature for 3 hours to carry out the activating reaction. This step yielded a suspension.
With 300 mg (0.52 mmol/g) of Fmoc-Cys(Trt)-(2-Cl)trityl resin as the starting material, somatostatin was synthesized by a standard method of solid phase Fmoc synthesis to yield H-Ala-Gly-Cys(Trt)-Lys(Boc)-Asn(Trt)-Phe-Phe-Trp(Boc)-Lys(Boc)-Thr(tBu)-Phe-Thr(tBu)-Ser(tBu)-Cys(Trt)-(2Cl)Trt resin.
2 mL of the suspension obtained above, 2 mL of N-methylpyrrolidone, and 100 µL of diisopropylamine were added thereto, and the mixture was stirred at room temperature for 1 hour. This reaction was carried out in five repeats, and it was confirmed that almost no unreacted amino groups were present on the solid phase resin by a ninhydrin reaction. The resin after the reaction was washed with methanol and dried under reduced pressure.
50 mg of the dry resin was treated with a solution containing 10 µL of triisopropylsilane, 25 µL of ethanedithiol, 25 µL of water, and 940 µL of trifluoroacetic acid at room temperature for 2 hours, and deprotection and cleavage from the resin were performed. After the reaction, a crude peptide was precipitated with 5 mL of cold ether; the precipitate was dissolved in an aqueous solution of acetonitrile and purified by reversed-phase HPLC. 0.5 mg of DOTA-somatostatin was successfully synthesized.
A mass analyzer was used to identify DOTA-somatostatin; it was confirmed that the molecular weight agreed with the calculated value. It was also confirmed that DOTA functions as a chelating agent as the positron-emitting metal nuclide 68Ga forms a complex with the DOTA-somatostatin obtained. It was also confirmed that DOTA-somatostatin possesses a function as somatostatin because positrons could be intensely detected in tumors that highly express somatostatin receptors in murines receiving an injection of DOTA-somatostatin having formed the complex of 68Ga.

### [Example 2]

DOTA-QBP1 (polyQ-binding peptide 1) was synthesized as described below.
With 370 mg (0.28 mmol/g) of NovaSyn (registered trademark) TGA resin as the starting material, 169 mg (0.41 mmol) of Fmoc-Asp(OtBu)-OH, 0.5M O-benzotriazolyl-N,N,N',N'-tetramethyluronium hexafluorophosphate (HBTU)/DMF 0.82 mL (0.41 mmol), 0.5M 1-hydroxybenzotriazole (HOBt)/DMF 0.82 mL (0.41 mmol), and 0.1 mg (0.8 µmol) of 4-(dimethylamino)pyridine were added thereto, and the mixture was stirred at room temperature for 1 hour. After the reaction, capping was performed with benzoic anhydride to yield 380 mg (0.24 mmol/g) of Fmoc-Asp(OtBu)-NovaSyn (registered trademark) TGA resin.
With 113 mg (0.24 mmol/g) of Fmoc-Asp(OtBu)-NovaSyn (registered trademark) TGA resin as the starting material, H-Ser(tBu)-Asn(Trt)-Trp-Lys(Boc)-Trp-Trp-Pro-Gly-Ile-Phe-Asp(OtBu)-NovaSyn (registered trademark) TGA resin was obtained by a standard Fmoc method. Next, 2 mg of succinimide and 4 mg of 1-ethyl-3-(dimethylaminopropyl)carbodiimide were dissolved in 2 mL of DMSO, 8 mg of 1,4,7,10-tetraazacyclododecane-N,N',N",N"'-tetraacetic acid (DOTA) was added thereto, and the mixture was stirred at room temperature for 3 hours to carry out the activating reaction. 2 mL of the above-described activating reaction liquid, 2 mL of N-methylpyrrolidone, and 100 µL of diisopropylamine were added to the resin obtained, and the mixture was stirred at room temperature for 1 hour. This reaction was carried out in five repeats; it was confirmed that almost no unreacted amino groups were present on the solid phase resin by a ninhydrin reaction. The resin after the reaction was washed with methanol and dried under reduced pressure to yield 160 mg of resin.
50 mg of the dry resin was treated with a solution containing 10 µL of triisopropylsilane, 25 µL of ethanedithiol, 25 µL of water, and 940 µL of trifluoroacetic acid at room temperature for 2 hours, and deprotection and cleavage from the resin were performed. After the reaction, a crude peptide was precipitated with 5 mL of cold ether; the precipitate was dissolved in an aqueous solution of acetonitrile and purified by reversed-phase HPLC. The peptide was identified as DOTA-QBP1 by mass analysis and amino acid analysis; 0.3 mg of the desired product was successfully synthesized.

### Industrial Applicability

The method of the present invention makes it possible to conveniently and efficiently introduce DOTA into a compound (e.g., peptides and the like) on a solid-phase carrier. In particular, in the method of the present invention, DOTA having no protecting group is used, so that the time required for final deprotection decreases significantly, compared with the conventional method, which involves the use of DOTA having a protecting group. Also, according to the method of the present invention, DOTA can easily be introduced into a peptide carried on a solid-phase carrier synthesized by solid phase peptide synthesis (SPPS), which is a standard technique in peptide synthesis, so that the present invention provides a highly practical method of synthesizing a DOTA-peptide conjugate. Furthermore, DOTA having no protecting group is much less expensive (about 1/10) compared with DOTA having a protecting group, allowing a significant cost reduction in the synthesis of DOTA-incorporating compounds.

This application is based on a patent application No. 2008-264520 filed in Japan (filing date: October 10, 2008), the contents of which are incorporated in full herein.

## Claims

1. A method of introducing 1,4,7,10-tetraazacyclododecane-N,N',N",N"'-tetraacetic acid (DOTA) into a compound, comprising a first step for preparing a mixed liquid of DOTA having no protecting group and dimethylsulfoxide, and a second step for contacting the mixed liquid with a compound carried on a solid-phase carrier.

2. The method according to claim 1, wherein the first step comprises a treatment for activating the DOTA using an activator.

3. The method according to claim 1 or 2, wherein the compound is a peptide or a polynucleotide.

4. The method according to claim 3, wherein the compound is a peptide.

5. The method according to claim 4, wherein the peptide is an antibody.

6. The method according to claim 4, wherein the peptide is at least one of somatostatin, secretin, octreotide, exenatide and QBP1.

7. The method according to claim 3, wherein the compound has been synthesized on the solid-phase carrier.

8. The method according to any one of claims 1 to 7, wherein the activator is 1-ethyl-3-(dimethylaminopropyl)carbodiimide.


SEQUENCE LISTING

<110>  RIKEN

<120>  Method for Introduction of DOTA

<130>  091464

<150>  JP2008-264520
<151>  2008-10-10

<160>  2

<170>  PatentIn version 3.5

<210>  1
<211>  14
<212>  PRT
<213>  Artificial Sequence

<220>
<223>  Sequence recited in Example 1


<220>
<221>  MOD_RES
<222>  (3)..(3)
<223>  Trt protection

<220>
<221>  MOD_RES
<222>  (4)..(4)
<223>  Boc protection

<220>
<221>  MOD_RES
<222>  (5)..(5)
<223>  Trt protection

<220>
<221>  MOD_RES
<222>  (8)..(8)
<223>  Boc protection

<220>
<221>  MOD_RES
<222>  (9)..(9)
<223>  Boc protection

<220>
<221>  MOD_RES
<222>  (10)..(10)
<223>  tBu protection

<220>
<221>  MOD_RES
<222>  (12)..(12)
<223>  tBu protection

<220>
<221>  MOD_RES
<222>  (13)..(13)
<223>  tBu protection

<220>
<221>  MOD_RES
<222>  (14)..(14)
<223>  Trt protection

<220>
<221>  MISC_FEATURE
<222>  (14)..(14)
<223>  Binding to (2Cl)Trt resin

<400>  1

Ala Gly Cys Lys Asn Phe Phe Trp Lys Thr Phe Thr Ser Cys
1               5                   10


<210>  2
<211>  11
<212>  PRT
<213>  Artificial Sequence

<220>
<223>  Sequence recited in Example 2


<220>
<221>  MOD_RES
<222>  (1)..(1)
<223>  tBu protection

<220>
<221>  MOD_RES
<222>  (2)..(2)
<223>  Trt protection

<220>
<221>  MOD_RES
<222>  (4)..(4)
<223>  Boc protection

<220>
<221>  MOD_RES
<222>  (11)..(11)
<223>  OtBu protection

<220>
<221>  MISC_FEATURE
<222>  (11)..(11)
<223>  Binding to NovaSyn (registered trademark) TGA resin

<400>  2

Ser Asn Trp Lys Trp Trp Pro Gly Ile Phe Asp
1               5                   10